# EUROPEAN PATENT APPLICATION

(11) **EP 4 663 125 A1**
(43) Date of publication of application: **17.12.2025**
(21) Application number: 23921473.7
(22) Date of filing: 31.10.2023
(51) Int. Cl.: A61B 5/346, A61B 5/00, G16H 50/20

(54) **METHOD FOR DETERMINING ANALYZABLE ELECTROCARDIOGRAM SECTION**

(30) Priority: 09.02.2023 KR 20230017457
(71) Applicant: VUNO INC., Seoul 06541 (KR)
(72) Inventor: JOO, Sunghoon, Seoul 04778 (KR); NA, Yeongyeon, Seoul 08026 (KR)
(74) Representative: Petraz, Gilberto Luigi
(86) International application number: PCT/KR2023/017124
(87) International publication number: WO 2024/167100

(57) **Abstract**

The present disclosure relates to a method for determining whether an electrocardiogram signal is readable, and more particularly, to a method for separating a diagnosable sub-interval from all intervals of an electrocardiogram signal by using a plurality of signal evaluation modules.

## Description

### [Technical Field]

The present disclosure relates to a method for determining whether an electrocardiogram signal is readable, and more particularly, to a method for separating a diagnosable sub-interval from all intervals of an electrocardiogram signal by using a plurality of signal assessment modules.

### [Background Art]

Electrocardiogram is one of the basic diagnostic methods for heart disease and is an important examination method for diagnosing cardiovascular diseases such as angina, myocardial infarction, and arrhythmia. The heart is a three-dimensional organ, and in order to grasp a pathological state of such an organ through an electrocardiogram, the electrocardiogram has been developed to obtain a lot of information by attaching several induction electrodes to the chest or attaching induction electrodes for a long period of time.

In recent years, wearable electrocardiographs (watch-type, patch-type, and pad-type) that improve measurement convenience have been developed, but most of the wearable electrocardiographs measure only a single induced signal and are performing measurement for a relatively short period of time. In particular, in the case of a wearable electrocardiogram device, a user who is not a medical staff mainly measures the electrocardiogram, but there is a problem in that there is an increasing probability of generation of noise due to low proficiency in measuring the electrocardiogram.

Therefore, there is a need to develop an algorithm that informs whether diagnosis or reading is possible even if some noise is generated when measuring the electrocardiogram.

### [Disclosure]

### [Technical Problem]

The present disclosure is contrived in response to the above-described background art, and an object of the present disclosure is to provide a method for determining whether an electrocardiogram signal is readable. For example, the present disclosure has been made in an effort to provide a method for determining whether an electrocardiogram signal is readable, which may separate an interval that is enabled to be sufficiently diagnosed by a disease diagnosis algorithm even if some noise is included in a measured electrocardiogram.

The technical problems of the present disclosure are not limited to the above-mentioned technical problems, and other technical problems not mentioned will be clearly understood by those skilled in the art from the following description.

### [Technical Solution]

In order to achieve the objects described above, a method for determining whether an electrocardiogram signal is readable, which is performed by a computing device is disclosed. The method may include: assessing a quality of the electrocardiogram signal by utilizing a plurality of signal assessment modules; and distinguishing a readable sub-interval and an unreadable sub-interval within an interval of the electrocardiogram signal based on assessment results of the plurality of signal assessment modules.

In an embodiment, the assessing of the quality of the electrocardiogram signal may include: generating a signal preprocessed based on the electrocardiogram signal by utilizing at least one preprocessing scheme; and generating a plurality of assessment results for the electrocardiogram signal by utilizing the preprocessed signal and the plurality of signal assessment modules.

In an embodiment, the plurality of respective signal assessment modules may include different noise reading functions.

In an embodiment, each of the plurality of signal assessment modules may identify a readable candidate sub-interval and an unreadable candidate sub-interval based on a predetermined threshold within the interval of the electrocardiogram signal.

In an embodiment, the threshold may be determined in response to the type of noise reading function.

In an embodiment, the distinguishing of the readable sub-interval and the unreadable sub-interval within the interval of the electrocardiogram signal based on the assessment results of the plurality of signal assessment modules may include identifying the readable sub-interval based on identifying an overlapping interval of a plurality of readable candidate sub-intervals identified by the plurality of signal assessment modules.

In an embodiment, the plurality of signal assessment modules may include at least one of a first type of signal assessment module configured to identify the readable candidate sub-interval or the unreadable candidate sub-interval based on a continuous assessment value for the electrocardiogram signal; or a second type of signal assessment module configured to identify the readable candidate sub-interval or the unreadable candidate sub-interval based on a predetermined segment-unit assessment value for the electrocardiogram signal.

In an embodiment, when a base assessment unit of the second type of signal assessment module is larger than the predetermined segment unit, the second type of signal assessment module may calculate the predetermined segment-unit assessment value by utilizing the base assessment-unit assessment value.

In an embodiment, when the base assessment unit of the second type of signal assessment module is larger than the predetermined segment unit, the second type of signal assessment module may perform an operation of obtaining assessment values of a plurality of base assessment units based on a plurality of assessment start points arranged at an interval of the predetermined segment unit; and an operation of calculating the assessment value of the predetermined segment unit by calculating representative values of the plurality of base assessment-unit assessment values at the interval of each predetermined segment unit.

In an embodiment, the method may further include at least one of inputting an entire interval or the readable sub-interval of the electrocardiogram signal into a disease analysis model when a longest interval of the readable sub-intervals is equal to or larger than a predetermined length; or determining re-measurement for the electrocardiogram signal when a longest interval of the readable sub-intervals is smaller than the predetermined length.

Further, in order to achieve the objects described above, a method for determining whether an electrocardiogram signal is re-measured, which is performed by a computing device, is disclosed. The method may include: distinguishing a readable sub-interval and an unreadable sub-interval within an interval of an electrocardiogram signal; and determining whether the electrocardiogram signal is re-measured based on the distinguished readable sub-interval.

Further, in order to achieve the objects described above, a computer program stored in a computer-readable storage medium is disclosed. When the computer program is executed by one or more processors, the computer program allows the one or more processors to perform the following operations for determining whether an electrocardiogram signal is readable, and the operations may include: an operation of assessing a quality of the electrocardiogram signal by utilizing a plurality of signal assessment modules; and an operation of distinguishing a readable sub-interval and an unreadable sub-interval within an interval of the electrocardiogram signal based on assessment results of the plurality of signal assessment modules.

Further, in order to achieve the objects described above, a computing device is disclosed. The computing device may include: at least one processor; and a memory, and the at least one processor may be configured to assess a quality of an electrocardiogram signal by utilizing a plurality of signal assessment modules, and distinguish a readable sub-interval and an unreadable sub-interval within an interval of the electrocardiogram signal based on assessment results of the plurality of signal assessment modules.

Technical solving means which can be obtained in the present disclosure are not limited to the aforementioned solving means and other unmentioned solving means will be clearly understood by those skilled in the art from the following description.

### [Advantageous Effects]

The present disclosure may provide a method that may specifically determine whether an electrocardiogram signal is readable. For example, the present disclosure may provide a method that may specifically identify readable sub-intervals within an interval of the electrocardiogram signal. Accordingly, the present disclosure may identify whether there are minimal sub-intervals that may be input by a disease analysis model, even if some noise is included in a measured electrocardiogram signal, and prevent unnecessary repeated measurements based on such identification.

Effects which can be obtained in the present disclosure are not limited to the aforementioned effects and other unmentioned effects will be clearly understood by those skilled in the art from the following description.

### [Description of Drawings]

Various aspects are now described with reference to the drawings and like reference numerals are generally used to designate like elements. In the following embodiments, for purposes of explanation, numerous specific details are set forth to provide a comprehensive understanding of one or more aspects. However, it will be apparent that the aspect(s) can be executed without the specific detailed matters. In other examples, known structures and apparatuses are illustrated in a block diagram form in order to facilitate description of the one or more aspects.
FIG. 1 is a block diagram for describing a computing device and learning data according to an embodiment of the present disclosure.
FIG. 2 is a conceptual diagram illustrating a neural network according to an embodiment of the present disclosure.
FIG. 3 illustrates a flowchart of a method for determining whether an electrocardiogram signal is readable according to an embodiment of the present disclosure.
FIG. 4 illustrates a conceptual diagram of a method for determining whether an electrocardiogram signal is readable according to an embodiment of the present disclosure.
FIG. 5 illustrates a flowchart of a method for determining whether an electrocardiogram signal is preprocessable and readable according to an embodiment of the present disclosure.
FIG. 6 illustrates a conceptual diagram of the method for determining whether the electrocardiogram signal is preprocessable and readable according to an embodiment of the present disclosure.
FIG. 7 illustrates a conceptual diagram illustrating the method for determining whether the electrocardiogram signal is readable based on a segment-unit assessment value according to an embodiment of the present disclosure.
FIG. 8 is a general schematic diagram of an exemplary computing environment in which embodiments of the present disclosure may be implemented.

### [Best Mode]

Various exemplary embodiments will now be described with reference to drawings. In the present specification, various descriptions are presented to provide appreciation of the present disclosure. However, it is apparent that the exemplary embodiments can be executed without the specific description.

"Component", "module", "system", and the like which are terms used in the specification refer to a computer-related entity, hardware, firmware, software, and a combination of the software and the hardware, or execution of the software. For example, the component may be a processing procedure executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

Suffixes "module" and "unit" for components used in the following description are given or mixed in consideration of easy preparation of the specification only and do not have their own distinguished meanings or roles.

Further, the terms "information" and "data" used in the specification may also be often used to be exchanged with each other.

It will be understood that when an element is referred to as being "connected" or "coupled" to another element, it can be directly connected or coupled to the other element or intervening elements may be present. In contrast, it should be understood that, when it is described that a component is "directly connected to" or "directly access" another component, no component is present there between.

The term "or" is intended to mean not exclusive "or" but inclusive "or". That is, when not separately specified or not clear in terms of a context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

It should be appreciated that the term "comprise" and/or "comprising" means presence of corresponding features and/or components. However, it should be appreciated that the term "comprises" and/or "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

Hereinafter, like reference numerals refer to like or similar elements regardless of reference numerals and a duplicated description thereof will be omitted. Further, in describing an embodiment disclosed in the present disclosure, a detailed description of related known technologies will be omitted if it is determined that the detailed description makes the gist of the embodiment of the present disclosure unclear. Further, the accompanying drawings are only for easily understanding the embodiment disclosed in this specification and the technical spirit disclosed by this specification is not limited by the accompanying drawings.

The term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

The description of the presented exemplary embodiments is provided so that those skilled in the art of the present disclosure use or implement the present disclosure. Various modifications to the exemplary embodiments will be apparent to those skilled in the art. Generic principles defined herein may be applied to other embodiments without departing from the scope of the present disclosure. Therefore, the present disclosure is not limited to the exemplary embodiments presented herein. The present disclosure should be analyzed within the widest range which is coherent with the principles and new features presented herein.

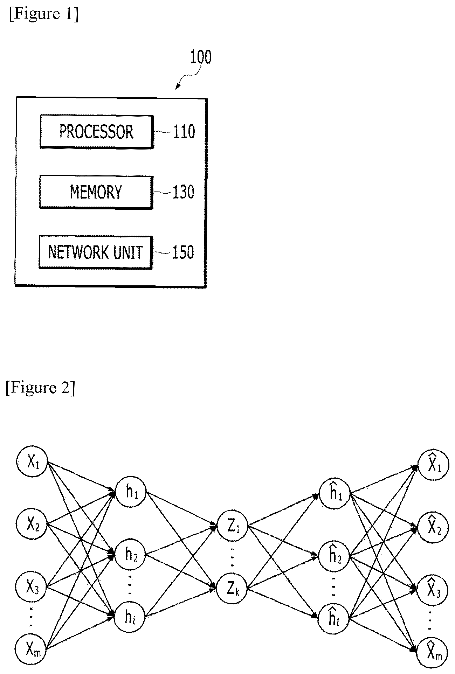

Hereinafter, a method for determining whether an electrocardiogram signal is readable according to the present disclosure will be described with reference to the matters illustrated in FIG. 1 to FIG. 8.

FIG. 1 is a block diagram for describing a computing device and learning data according to an embodiment of the present disclosure.

A configuration of the computing device 100 illustrated in FIG. 1 is only an example shown through simplification. In an exemplary embodiment of the present disclosure, the computing device 100 may include other components for performing a computing environment of the computing device 100 and only some of the disclosed components may constitute the computing device 100.

The computing device 100 may include a processor 110, a memory 130, and a network unit 150. The processor 110 may be constituted by one or more cores and may include processors for data analysis and deep learning, which include a central processing unit (CPU), a general purpose graphics processing unit (GPGPU), a tensor processing unit (TPU), and the like of the computing device. The processor 110 may read a computer program stored in the memory 130 to perform data processing for machine learning according to an exemplary embodiment of the present disclosure. According to an exemplary embodiment of the present disclosure, the processor 110 may perform a calculation for learning the neural network. The processor 110 may perform calculations for learning the neural network, which include processing of input data for learning in deep learning (DL), extracting a feature in the input data, calculating an error, updating a weight of the neural network using backpropagation, and the like. At least one of the CPU, GPGPU, and TPU of the processor 110 may process learning of a network function. For example, both the CPU and the GPGPU may process the learning of the network function and data classification using the network function. Further, in an exemplary embodiment of the present disclosure, processors of a plurality of computing devices may be used together to process the learning of the network function and the data classification using the network function. Further, the computer program executed in the computing device according to an exemplary embodiment of the present disclosure may be a CPU, GPGPU, or TPU executable program.

According to an exemplary embodiment of the present disclosure, the memory 130 may store any type of information generated or determined by the processor 110 and any type of information received by the network unit 150.

According to an exemplary embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

The network unit 150 according to several embodiments of the present disclosure may use various wired communication systems, such as a Public Switched Telephone Network (PSTN), an x Digital Subscriber Line (xDSL), a Rate Adaptive DSL (RADSL), a Multi Rate DSL (MDSL), a Very High Speed DSL (VDSL), a Universal Asymmetric DSL (UADSL), a High Bit Rate DSL (HDSL), and a local area network (LAN).

The network unit 150 presented in the present specification may use various wireless communication systems, such as Code Division Multi Access (CDMA), Time Division Multi Access (TDMA), Frequency Division Multi Access (FDMA), Orthogonal Frequency Division Multi Access (OFDMA), Single Carrier-FDMA (SC-FDMA), and other systems.

In the present disclosure, the network unit 150 may be configured regardless of a communication aspect, such as wired communication and wireless communication, and may be configured by various communication networks, such as a Personal Area Network (PAN) and a Wide Area Network (WAN). Further, the network may be a publicly known World Wide Web (WWW), and may also use a wireless transmission technology used in short range communication, such as Infrared Data Association (IrDA) or Bluetooth.

The techniques described herein may be used in other networks in addition to those mentioned above.

The processor 110 or the memory 130 may obtain an electrocardiogram signal from an electrocardiogram device. The obtaining may be through the network unit 150 or directly connected to the electrocardiogram device. The electrocardiogram device may be included without limitation as long as it is a device capable of measuring electrocardiogram. As an example, the electrocardiogram device may be a device that measures electrocardiogram for a short period of time within one minute with a smartphone, a smartwatch, a portable patch, a portable pad, other portable, dedicated electrocardiogram measuring device, or combinations thereof. As another example, the electrocardiogram device may be a standard 12-lead electrocardiogram device capable of short-term electrocardiogram recording. In this case, even if the electrocardiogram for the short period of time includes some noise, according to the present disclosure, an interval that may be sufficiently diagnosed by a disease diagnosis algorithm may be separated, and an electrocardiogram device user may check a disease diagnosis result without unnecessary repeated measurement.

According to an embodiment of the present disclosure, the memory 130 may store data related to the electrocardiogram signal. Further, the network unit may transmit the data, and the processor 110 may perform operations for determining, based on the electrocardiogram signal, whether the electrocardiogram signal is readable.

FIG. 2 is a schematic diagram illustrating a network function according to an exemplary embodiment of the present disclosure.

Throughout the present specification, a computation model, the neural network, a network function, and the neural network may be used as the same meaning. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

In the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, a definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which do not have other input nodes connected through the links. Similarly thereto, the final output node may mean one or more nodes which do not have the output node in the relationship with other nodes among the nodes in the neural network. Further, a hidden node may mean nodes constituting the neural network other than the initial input node and the final output node.

In the neural network according to an exemplary embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another exemplary embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another exemplary embodiment of the present disclosure, the number of nodes of the input layer may be larger than the number of nodes of the output layer, and the neural network may be a neural network of a type in which the number of nodes increases from the input layer to the hidden layer. The neural network according to still yet another exemplary embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an auto encoder, generative adversarial networks (GAN), a restricted Boltzmann machine (RBM), a deep belief network (DBN), a Q network, a U network, a Siam network, a Generative Adversarial Network (GAN), and the like. The description of the deep neural network described above is just an example and the present disclosure is not limited thereto.

In an exemplary embodiment of the present disclosure, the network function may include the auto encoder. The auto encoder may be a kind of artificial neural network for outputting output data similar to input data. The auto encoder may include at least one hidden layer and odd hidden layers may be disposed between the input and output layers. The number of nodes in each layer may be reduced from the number of nodes in the input layer to an intermediate layer called a bottleneck layer (encoding), and then expanded symmetrical to reduction to the output layer (symmetrical to the input layer) in the bottleneck layer. The auto encoder may perform non-linear dimensional reduction. The number of input and output layers may correspond to a dimension after preprocessing the input data. The auto encoder structure may have a structure in which the number of nodes in the hidden layer included in the encoder decreases as a distance from the input layer increases. When the number of nodes in the bottleneck layer (a layer having a smallest number of nodes positioned between an encoder and a decoder) is too small, a sufficient amount of information may not be delivered, and as a result, the number of nodes in the bottleneck layer may be maintained to be a specific number or more (e.g., half of the input layers or more).

The neural network may be learned in at least one scheme of supervised learning, unsupervised learning, semi supervised learning, or reinforcement learning. The learning of the neural network may be a process in which the neural network applies knowledge for performing a specific operation to the neural network.

The neural network may be learned in a direction to minimize errors of an output. The learning of the neural network is a process of repeatedly inputting training data into the neural network and calculating the output of the neural network for the training data and the error of a target and back-propagating the errors of the neural network from the output layer of the neural network toward the input layer in a direction to reduce the errors to update the weight of each node of the neural network. In the case of the supervised learning, the training data labeled with a correct answer is used for each training data (i.e., the labeled training data) and in the case of the unsupervised learning, the correct answer may not be labeled in each training data. That is, for example, the training data in the case of the supervised learning related to the data classification may be data in which category is labeled in each training data. The labeled training data is input to the neural network, and the error may be calculated by comparing the output (category) of the neural network with the label of the training data. As another example, in the case of the unsupervised learning related to the data classification, the training data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the back propagation.

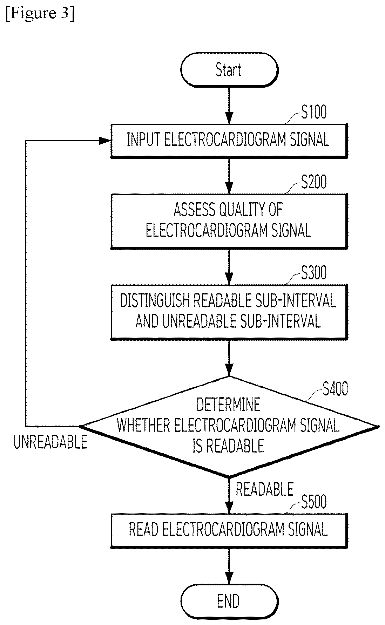

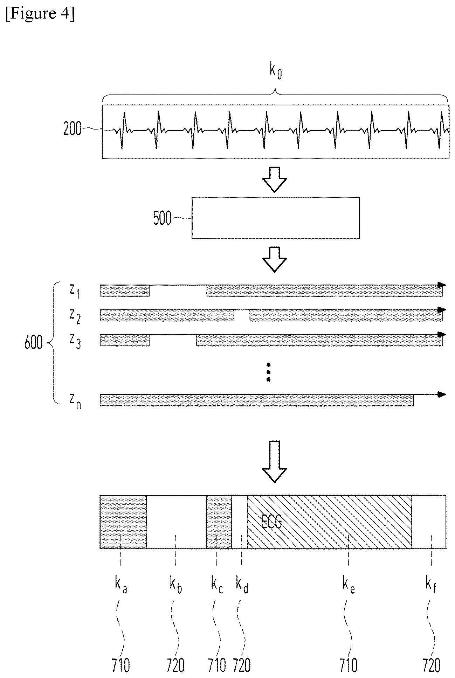

FIGS. 3 and 4 illustrate a flowchart and a conceptual diagram of a method for determining whether an electrocardiogram signal is readable according to an embodiment of the present disclosure.

Referring to FIGS. 3 and 4, a method for determining whether an electrocardiogram signal 200 is readable, which is performed by a computing device according to an embodiment of the present disclosure, may include a step S100 of receiving an electrocardiogram signal 200 input by an electrocardiogram device, a step S200 of evaluating a quality of the electrocardiogram signal 200 by utilizing a plurality of signal assessment modules 500, and a step S300 of distinguishing a readable sub-interval 710 and an unreadable sub-interval 720 within an entire interval k0 of the electrocardiogram signal by utilizing the plurality of signal assessment modules 500.

In step S100 above, first, the electrocardiogram signal 200 is measured by the electrocardiogram device. Here, a minimum length of the entire interval k0 of the electrocardiogram signal may be specified by a user or may be determined according to the type of electrocardiogram measurement equipment. In an embodiment, the computing device 100 may be directly connected to the electrocardiogram device through the network unit 150 to receive the electrocardiogram signal 200, or may directly obtain the electrocardiogram signal 200 as the electrocardiogram device itself. In addition, the computing device 100 may obtain the electrocardiogram signal 200 pre-stored in the memory 130.

In step S200 above, the computing device 100 may obtain a plurality of assessment results 600 for the electrocardiogram signal 200 by utilizing the plurality of signal assessment modules 500, and may evaluate the quality of the electrocardiogram signal 200 based on the plurality of assessment results 600.

The electrocardiogram device may include a portable electrocardiogram device that may be used by a non-professional, and when the non-professional measures the electrocardiogram signal, there are many factors that make it difficult to read the electrocardiogram signal 200, such as noise, compared to when a skilled medical staff measures the electrocardiogram signal, and thus it is difficult to obtain a relatively high-quality electrocardiogram signal 200. Accordingly, the computing device 100 of the present disclosure may introduce the plurality of signal assessment modules 500 to accurately evaluate the quality of the input electrocardiogram signal.

Furthermore, a method for evaluating the quality of the electrocardiogram signal is not presented as a single standard, and the introduction of the plurality of signal assessment modules 500 may complement limitations and maximize advantages of individual assessment methods. In addition, the method for evaluating the quality of the electrocardiogram signal has been newly and steadily developed, and for the plurality of signal assessment modules 500, it is possible to add or update some modules for improving performance.

In an embodiment, the plurality of assessment modules 500 may generate assessment results z1, z2, z3, ..., zn that distinguish between a "readable candidate sub-interval" and a "unreadable candidate sub-interval" within the entire interval k0 of the electrocardiogram signal. In addition, the computing device 100 may preprocess the electrocardiogram signal 200 in at least one scheme, and then input the preprocessed electrocardiogram signal to the plurality of signal assessment modules 500. Here, the processing scheme may be a plurality of preprocessing schemes different from each other according to the plurality of signal assessment modules 500.

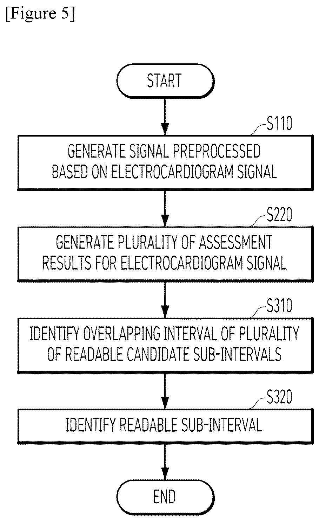

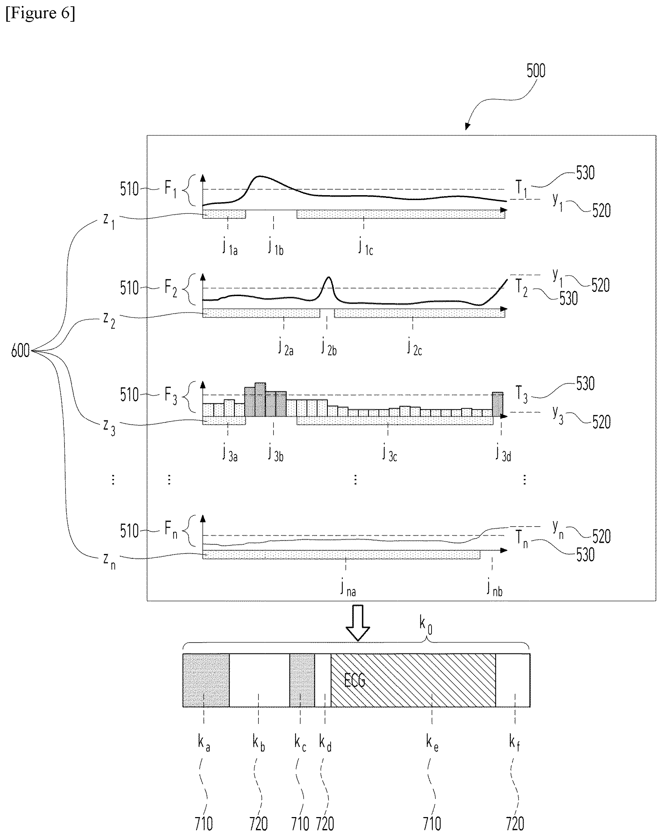

Details related to the plurality of signal assessment modules 500 (e.g., details related to the readable candidate sub-interval, the unreadable candidate-sub-interval, the different schemes of preprocessing, and the like) will be described below with reference to FIGS. 5 and 6.

In step S300 above, the computing device 100 may distinguish a "readable sub-interval 710" and a "unreadable sub-interval 720" within the entire interval k0 of the electrocardiogram signal based on the "readable candidate sub-intervals" included in the plurality of assessment results 600. As an example, the computing device 100 may identify the "readable sub-interval 710" based on identifying an overlapping interval of the "readable candidate sub-intervals" included in the plurality of assessment results 600. Meanwhile, a more detailed embodiment related to distinguishing the "readable sub-interval 710" and the "unreadable sub-interval 720" by the computing device 100 will be described below with reference to FIGS. 5 and 6.

Additionally, the method for determining whether the electrocardiogram signal 200 is readable may further include a step S400 of determining, based on the readable sub-interval 710, whether the electrocardiogram signal 200 is readable.

In step S400 above, the computing device 100 may determine that the electrocardiogram signal 200 is readable when a longest interval among the readable sub-intervals 710 is greater than or equal to a predetermined length. On the other hand, the computing device 100 may determine that the electrocardiogram signal 200 is unreadable when a longest interval among the readable sub-intervals 710 is smaller than the predetermined length. According to the method for determining whether the electrocardiogram signal 200 is readable, even if noise is included in the measured electrocardiogram signal 200 in some degree, an interval that may be sufficiently diagnosed by the disease diagnosis algorithm may be separated, so that a user may confirm the disease diagnosis result without unnecessary repeated measurement of the electrocardiogram.

The predetermined length may be set to a length sufficient to provide diagnostic information with a high level of accuracy through the electrocardiogram signal 200, and the level of accuracy may be set differently according to a desired diagnosis. As an example, when the accuracy level is related to high-risk disease information such as stroke, the accuracy level is set higher than when the number of frequent measurements is related to prediction of a chronic disease prognosis, so that a possibility of determining false negative for determining whether the stroke occurs may be reduced.

The method for determining whether the electrocardiogram signal 200 is readable according to the present disclosure may include a step S500 of reading the electrocardiogram signal 200 when it is determined that the electrocardiogram signal 200 is readable. As an example, in step S500 above, the computing device 100 may read the entire interval k0 or the readable sub-interval 710 of the electrocardiogram signal. As another example, the computing device 100 may transmit information about the entire interval k0 or the readable sub-interval 710 of the electrocardiogram signal to a server such as a medical institution through the network unit 150 to read the electrocardiogram signal 200. The reading may be performed by a disease analysis model utilizing any one or more of a rule based system or artificial intelligence, depending on the purpose.

On the other hand, the method may include a step of determining electrocardiogram re-measurement when it is determined that the electrocardiogram signal 200 is unreadable. When the re-measurement is determined, the computing device 100 may send, to the user, a signal indicating that the electrocardiogram re-measurement is required by an alarm or the like, or may automatically repeat the re-measurement in the electrocardiogram device. The signal may be, for example, a visual expression such as a letter or a symbol, or may be included without limitation in its form such as sound or vibration.

FIGS. 5 and 6 illustrate a flowchart and a conceptual diagram of a method for determining whether an electrocardiogram signal is preprocessed and readable according to an embodiment of the present disclosure.

According to FIGS. 5 and 6, the step of assessing the quality of the electrocardiogram signal 200 by utilizing the plurality of signal assessment modules 500 may include a step S210 of generating a preprocessed signal based on the electrocardiogram signal 200, and a step S220 of generating a plurality of assessment results 600 for the electrocardiogram signal 200.

In step S210, the processor 110 may perform preprocessing by changing a sampling frequency or applying a bandpass filter with respect to the electrocardiogram signal 200. Here, the preprocessing scheme may be controlled to fit a noise read function (e.g., F1, F2, F3, ..., Fn) to be input.

In step S220 above, each of the plurality of signal assessment modules 500 for the electrocardiogram signal 200 may receive the preprocessed signal and input the received signal into a plurality of noise reading functions 510 to obtain a plurality of assessment values 520. Here, the noise reading functions 510 may be included in each of the plurality of signal assessment modules 500 one by one. In the electrocardiogram signal 200, the presence of noise is a major factor that interferes with accurate sensing of important clinical characteristics and degrades the quality of the electrocardiogram signal 200. Therefore, by introducing a different noise reading function 510 to each of the plurality of signal assessment modules 500, various noises in the electrocardiogram signal 200 may be searched, and intervals such as the unreadable sub-interval 720 containing noise may be excluded from a reading process of the electrocardiogram signal 200.

In addition, the plurality of signal assessment modules 500 may compare the obtained plurality of assessment values 520 with a plurality of thresholds 530 to obtain a plurality of assessment results 600 for the entire interval k0 of the electrocardiogram signal.

As an example, the computing device 100 preprocesses the electrocardiogram signal 200 to obtain respective (n) signals constituting the preprocessed signal. Next, for each of the n signals, each of the n functions F1, F2, F3, ..., Fn constituting the plurality of noise reading functions 510 may be input to obtain each of the n assessment values y1, y2, y3, ..., yn constituting the plurality of assessment values 520. Here, each of the respective (n) functions F1, F2, F3, ..., Fn may output the respective (n) assessment values yl, y2, y3, ..., yn in parallel. In addition, the respective (n) functions F1, F2, F3, ...., Fn may define an analyzable electrocardiogram segment by combining various applicable signal quality assessment functions, and may freely add or exclude signal quality assessment functions through the combination.

Next, the computing device 100 may identify, within the interval of the electrocardiogram signal, a readable candidate sub-interval and an unreadable candidate-sub-interval based on a predetermined threshold. As an example, the computing device 100 compares the respective (n) assessment values y 1, y2, y3, ..., and yn with respective (n) thresholds T1, T2, T3, ..., Tn constituting the plurality of thresholds 530 to obtain assessment results z1, z2, z3, ...., zn. Here, when the assessment value is less than or equal to a threshold, the electrocardiogram signal 200 may be distinguished by a readable candidate sub-interval (e.g., j1a, jlc) corresponding to a readable quality, and when the assessment value exceeds the threshold, the electrocardiogram signal 200 may be distinguished by an unreadable candidate- sub-interval (i.e., jlb) corresponding to an unreadable quality. Alternatively, when the assessment value is less than the threshold, the electrocardiogram signal 200 may be distinguished by a readable candidate sub-interval corresponding to a readable quality, and when the assessment value is equal to or more than the threshold, the electrocardiogram signal 200 may be distinguished by an unreadable candidate- sub-interval corresponding to an unreadable quality.

As an example, the j1a and j1c intervals in which the assessment value y1 derived by the noise reading function F1 is less than or equal to the threshold T1 among the entire interval k0 of the electrocardiogram signal may be distinguished as readable candidate sub-intervals. On the other hand, the jlb intervals in which the assessment value y1 derived by the noise reading function F1 exceeds the threshold T1 (equal to or greater than the threshold T1) may be distinguished as unreadable candidate sub-intervals.

Next, the method may further include a step of identifying the readable sub-interval 710 by determining an overlapping interval of the plurality of readable candidate sub-intervals identified by the plurality of signal assessment modules 500. The overlapping section may be an interval defined as a readable interval by combining the assessment results for the electrocardiogram signal 200. As an example, first, the noise reading functions F1 to Fn are respectively utilized to obtain z1, z2, z3, ..., zn corresponding to the assessment results. Next, the j1a, j1c, j2a, j2c, j3a, j3c, ..., jna intervals corresponding to the readable candidate sub-interval among the assessment results is identified. Finally, in the identified readable candidate sub-interval, the ka, kc, and ke intervals corresponding to the overlapping interval may be identified as the readable sub-interval 710.

The plurality of signal assessment modules 500 may include a first type of signal assessment module that identifies the readable candidate sub-interval or the unreadable candidate sub-interval based on a continuous assessment value for the electrocardiogram signal 200. As an example, F1, F2, and Fn of a plurality of noise reading functions 510 included in the plurality of signal assessment modules 500 may obtain the readable candidate sub-intervals, such as generating y1, y2, and yn of a plurality of assessment values 520, which may be represented as a continuous function over time, and obtaining j1a and j1c based on the y1 and a threshold T1, j2a, and j2c based on the y2 and a threshold T2, and jna based on the yn and a threshold Tn.

The plurality of signal assessment modules 500 may include a second type of signal assessment module that identifies the readable candidate sub-interval or the unreadable candidate sub-interval based on a predetermined segment-unit assessment value for the electrocardiogram signal 200. As an example, when a predetermined segment unit of the second type of signal assessment module is set to 1 second, during k0 seconds, which is an entire interval of the electrocardiogram signal, the second type of signal assessment module generates k0 assessment values, and identifies the readable candidate sub-interval or the unreadable candidate sub-interval based on the k0 assessment values. As an example, for F3 of the plurality of noise read functions 510 included in the plurality of signal assessment modules 500, y3 that may be represented as a continuous function over time among the plurality of assessment values 520 may be obtained, and the readable candidate sub-intervals may be obtained, such as obtaining j3a and j3c based on the y3 and the threshold T3.

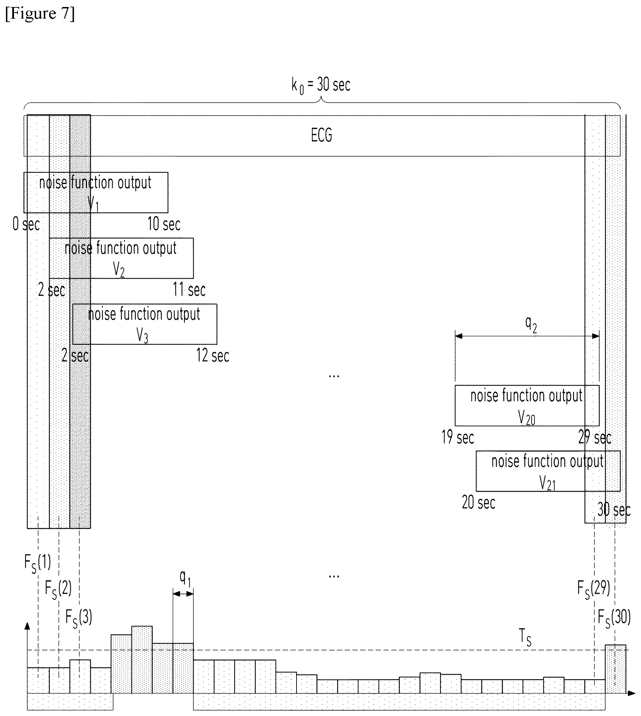

Here, an embodiment of obtaining assessment values for a predetermined segment in the second type of signal assessment module will be described below in FIG. 7.

FIG. 7 illustrates a conceptual diagram illustrating the method for determining whether the electrocardiogram signal is readable based on a segment-unit evaluation value according to an embodiment of the present disclosure.

According to FIG. 7, when a base assessment unit q2 of the second type of signal assessment module is larger than the predetermined segment unit q1, the second type of signal assessment module may calculate assessment values Fs(1), Fs(2), Fs(3), ..., Fs(29), and Fs(30) at the predetermined segment unit (q1) interval by utilizing assessment values v1, v2, v3, ..., v20, and v21 corresponding to the base assessment unit q2.

As an example, the method for calculating the assessment values Fs(1), Fs(2), Fs(3), ..., Fs(29), and Fs(30) may include an operation of obtaining assessment values v1, v2, v3, ..., v20, and v21 of the plurality of base assessment units q2 based on a plurality of assessment start points arranged at intervals of the predetermined segment unit q1. Further, an operation of calculating the assessment value of the predetermined segment unit q1 may be performed by calculating representative values Fs (1), Fs (2), Fs (3), Fs (29), and Fs (30) of the assessment values v1, v2, v3, ..., v20, and v21 of the plurality of basic assessment units at intervals of the respective predetermined segment units q1.

As an example, when the entire interval k0 of the electrocardiogram signal is 30 seconds, the predetermined segment unit q1 is 1 second, and the base assessment unit q2 is 10 seconds, the assessment value of 0 to 1 second after the start of electrocardiogram measurement is one v1, which corresponds to the representative value Fs(1). Next, the assessment values of 1 to 2 seconds after the start of the electrocardiogram measurement are two, i.e., v1 and v2, and when the representative value Fs(2) is set to a mean value of the assessment values, Fs(2) = (v1 + v2)/2 may be expressed. The noise assessment values v1, v2, v3, ..., v20, and v21 are obtained by moving the predetermined segment unit q1 for 1 second in the same scheme as described above. Here, a movement of the segment unit q1 occurs in the entire interval k0 of the electrocardiogram signal. Next, representative values Fs(1), Fs(2), Fs(3), ..., Fs(29), and Fs(30) may be defined by obtaining a mean between valid assessment values at intervals of 1 second, which is the predetermined segment unit q1. As a result, even when the base assessment unit q2 required to calculate the noise assessment values v1, v2, v3, ..., v20, and v21 is large, representative values Fs(1), Fs(2), Fs(3), ..., Fs(29), Fs(30) may be obtained for each predetermined segment unit q1, and a readable sub-interval of a sufficient resolution for confirming the quality of the electrocardiogram signal may be obtained. As an example, when a Gaussian noise function is used to perform the assessment on the electrocardiogram signal 200, representative values are obtained at predetermined intervals of segment unit q1 irrespective of the base assessment unit q2 of the function, and the electrocardiogram signal 200 may be assessed.

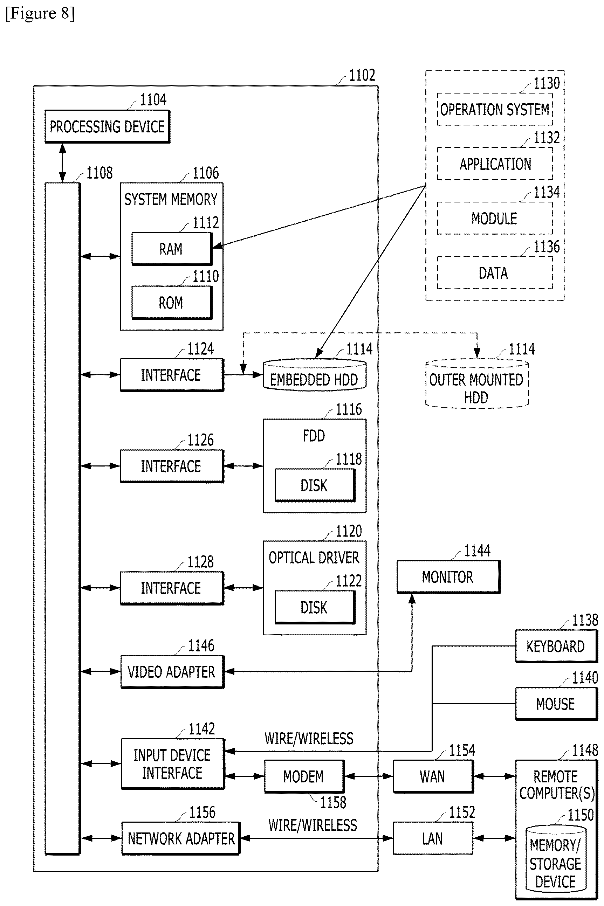

FIG. 8 is a general schematic diagram of an exemplary computing environment in which embodiments of the present disclosure may be implemented.

Referring to the description of FIG. 8, as a computer program stored in a computer-readable storage medium, the computer program, when executed by one or more processors, causes the one or more processors to perform operations of generating learning data for electrocardiogram diagnosis, and the operations may include an operation of segmenting the electrocardiogram data to obtain a plurality of electrocardiogram segments (ECG segments), classifying a type of each of the plurality of electrocardiogram segments based on at least one of a signal quality assessment or an electrocardiogram abnormality assessment, and an operation of generating learning data for the electrocardiogram diagnosis based on the classified type of each of the plurality of electrocardiogram segments.

In the meantime, according to an embodiment of the present disclosure, a computer readable medium storing a data structure is disclosed.

The data structure may refer to organization, management, and storage of data that enable efficient access and modification of data. The data structure may refer to organization of data for solving a specific problem (for example, data search, data storage, and data modification in the shortest time). The data structure may also be defined with a physical or logical relationship between the data elements designed to support a specific data processing function. A logical relationship between data elements may include a connection relationship between user defined data elements. A physical relationship between data elements may include an actual relationship between the data elements physically stored in a computer readable storage medium (for example, a permanent storage device). In particular, the data structure may include a set of data, a relationship between data, and a function or a command applicable to data. Through the effectively designed data structure, the computing device may perform a calculation while minimally using resources of the computing device. In particular, the computing device may improve efficiency of calculation, reading, insertion, deletion, comparison, exchange, and search through the effectively designed data structure.

The data structure may be divided into a linear data structure and a non-linear data structure according to the form of the data structure. The linear data structure may be the structure in which only one data is connected after one data. The linear data structure may include a list, a stack, a queue, and a deque. The list may mean a series of dataset in which order exists internally. The list may include a linked list. The linked list may have a data structure in which data is connected in a method in which each data has a pointer and is linked in a single line. In the linked list, the pointer may include information about the connection with the next or previous data. The linked list may be expressed as a single linked list, a double linked list, and a circular linked list according to the form. The stack may have a data listing structure with limited access to data. The stack may have a linear data structure that may process (for example, insert or delete) data only at one end of the data structure. The data stored in the stack may have a data structure (Last In First Out, LIFO) in which the later the data enters, the sooner the data comes out. The queue is a data listing structure with limited access to data, and may have a data structure (First In First Out, FIFO) in which the later the data is stored, the later the data comes out, unlike the stack. The deque may have a data structure that may process data at both ends of the data structure.

The non-linear data structure may be the structure in which the plurality of data is connected after one data. The non-linear data structure may include a graph data structure. The graph data structure may be defined with a vertex and an edge, and the edge may include a line connecting two different vertexes. The graph data structure may include a tree data structure. The tree data structure may be the data structure in which a path connecting two different vertexes among the plurality of vertexes included in the tree is one. That is, the tree data structure may be the data structure in which a loop is not formed in the graph data structure.

Throughout the present specification, a calculation model, a nerve network, the network function, and the neural network may be used with the same meaning. Hereinafter, the terms of the calculation model, the nerve network, the network function, and the neural network are unified and described with a neural network. The data structure may include a neural network. Further, the data structure including the neural network may be stored in a computer readable medium. The data structure including the neural network may also include preprocessed data for processing by the neural network, data input to the neural network, a weight of the neural network, a hyper-parameter of the neural network, data obtained from the neural network, an active function associated with each node or layer of the neural network, and a loss function for training of the neural network. The data structure including the neural network may include predetermined configuration elements among the disclosed configurations. That is, the data structure including the neural network may include the entirety or a predetermined combination of pre-processed data for processing by neural network, data input to the neural network, a weight of the neural network, a hyper parameter of the neural network, data obtained from the neural network, an active function associated with each node or layer of the neural network, and a loss function for training the neural network. In addition to the foregoing configurations, the data structure including the neural network may include predetermined other information determining a characteristic of the neural network. Further, the data structure may include all type of data used or generated in a computation process of the neural network, and is not limited to the foregoing matter. The computer readable medium may include a computer readable recording medium and/or a computer readable transmission medium. The neural network may be formed of a set of interconnected calculation units which are generally referred to as "nodes". The "nodes" may also be called "neurons." The neural network consists of one or more nodes.

The data structure may include data input to the neural network. The data structure including the data input to the neural network may be stored in the computer readable medium. The data input to the neural network may include training data input in the training process of the neural network and/or input data input to the training completed neural network. The data input to the neural network may include data that has undergone pre-processing and/or data to be pre-processed. The pre-processing may include a data processing process for inputting data to the neural network. Accordingly, the data structure may include data to be pre-processed and data generated by the pre-processing. The foregoing data structure is merely an example, and the present disclosure is not limited thereto.

The data structure may include a weight of the neural network (in the present specification, weights and parameters may be used with the same meaning), Further, the data structure including the weight of the neural network may be stored in the computer readable medium. The neural network may include a plurality of weights. The weight is variable, and in order for the neural network to perform a desired function, the weight may be varied by a user or an algorithm. For example, when one or more input nodes are connected to one output node by links, respectively, the output node may determine a data value output from the output node based on values input to the input nodes connected to the output node and the weight set in the link corresponding to each of the input nodes. The foregoing data structure is merely an example, and the present disclosure is not limited thereto.

For a non-limited example, the weight may include a weight varied in the neural network training process and/or the weight when the training of the neural network is completed. The weight varied in the neural network training process may include a weight at a time at which a training cycle starts and/or a weight varied during a training cycle. The weight when the training of the neural network is completed may include a weight of the neural network completing the training cycle. Accordingly, the data structure including the weight of the neural network may include the data structure including the weight varied in the neural network training process and/or the weight when the training of the neural network is completed. Accordingly, it is assumed that the weight and/or a combination of the respective weights are included in the data structure including the weight of the neural network. The foregoing data structure is merely an example, and the present disclosure is not limited thereto.

The data structure including the weight of the neural network may be stored in the computer readable storage medium (for example, a memory and a hard disk) after undergoing a serialization process. The serialization may be the process of storing the data structure in the same or different computing devices and converting the data structure into a form that may be reconstructed and used later. The computing device may serialize the data structure and transceive the data through a network. The serialized data structure including the weight of the neural network may be reconstructed in the same or different computing devices through deserialization. The data structure including the weight of the neural network is not limited to the serialization. Further, the data structure including the weight of the neural network may include a data structure (for example, in the non-linear data structure, B-Tree, Trie, m-way search tree, AVL tree, and Red-Black Tree) for improving efficiency of the calculation while minimally using the resources of the computing device. The foregoing matter is merely an example, and the present disclosure is not limited thereto.

The data structure may include a hyper-parameter of the neural network. The data structure including the hyper-parameter of the neural network may be stored in the computer readable medium. The hyper-parameter may be a variable varied by a user. The hyper-parameter may include, for example, a learning rate, a cost function, the number of times of repetition of the training cycle, weight initialization (for example, setting of a range of a weight value to be weight-initialized), and the number of hidden units (for example, the number of hidden layers and the number of nodes of the hidden layer). The foregoing data structure is merely an example, and the present disclosure is not limited thereto.

The present disclosure has been described as being generally implementable by the computing device, but those skilled in the art will appreciate well that the present disclosure is combined with computer executable commands and/or other program modules executable in one or more computers and/or be implemented by a combination of hardware and software.

In general, a program module includes a routine, a program, a component, a data structure, and the like performing a specific task or implementing a specific abstract data form. Further, those skilled in the art will well appreciate that the method of the present disclosure may be carried out by a personal computer, a hand-held computing device, a microprocessor-based or programmable home appliance (each of which may be connected with one or more relevant devices and be operated), and other computer system configurations, as well as a single-processor or multiprocessor computer system, a mini computer, and a main frame computer.

The embodiments of the present disclosure may be carried out in a distribution computing environment, in which certain tasks are performed by remote processing devices connected through a communication network. In the distribution computing environment, a program module may be located in both a local memory storage device and a remote memory storage device.

The computer generally includes various computer readable media. The computer accessible medium may be any type of computer readable medium, and the computer readable medium includes volatile and non-volatile media, transitory and non-transitory media, and portable and non-portable media. As a non-limited example, the computer readable medium may include a computer readable storage medium and a computer readable transport medium. The computer readable storage medium includes volatile and non-volatile media, transitory and non-transitory media, and portable and non-portable media constructed by a predetermined method or technology, which stores information, such as a computer readable command, a data structure, a program module, or other data. The computer readable storage medium includes a RAM, a Read Only Memory (ROM), an Electrically Erasable and Programmable ROM (EEPROM), a flash memory, or other memory technologies, a Compact Disc (CD)-ROM, a Digital Video Disk (DVD), or other optical disk storage devices, a magnetic cassette, a magnetic tape, a magnetic disk storage device, or other magnetic storage device, or other predetermined media, which are accessible by a computer and are used for storing desired information, but is not limited thereto.

The computer readable transport medium generally implements a computer readable command, a data structure, a program module, or other data in a modulated data signal, such as a carrier wave or other transport mechanisms, and includes all of the information transport media. The modulated data signal means a signal, of which one or more of the characteristics are set or changed so as to encode information within the signal. As a non-limited example, the computer readable transport medium includes a wired medium, such as a wired network or a direct-wired connection, and a wireless medium, such as sound, Radio Frequency (RF), infrared rays, and other wireless media. A combination of the predetermined media among the foregoing media is also included in a range of the computer readable transport medium.

An illustrative environment 1100 including a computer 1102 and implementing several aspects of the present disclosure is illustrated, and the computer 1102 includes a processing device 1104, a system memory 1106, and a system bus 1108. The system bus 1108 connects system components including the system memory 1106 (not limited) to the processing device 1104. The processing device 1104 may be a predetermined processor among various commonly used processors. A dual processor and other multi-processor architectures may also be used as the processing device 1104.

The system bus 1108 may be a predetermined one among several types of bus structure, which may be additionally connectable to a local bus using a predetermined one among a memory bus, a peripheral device bus, and various common bus architectures. The system memory 1106 includes a ROM 1110, and a RAM 1112. A basic input/output system (BIOS) is stored in a non-volatile memory 1110, such as a ROM, an EPROM, and an EEPROM, and the BIOS includes a basic routing helping a transport of information among the constituent elements within the computer 1102 at a time, such as starting. The RAM 1112 may also include a high-rate RAM, such as a static RAM, for caching data.

The computer 1102 also includes an embedded hard disk drive (HDD) 1114 (for example, enhanced integrated drive electronics (EIDE) and serial advanced technology attachment (SATA)) - the embedded HDD 1114 being configured for exterior mounted usage within a proper chassis (not illustrated) - a magnetic floppy disk drive (FDD) 1116 (for example, which is for reading data from a portable diskette 1118 or recording data in the portable diskette 1118), and an optical disk drive 1120 (for example, which is for reading a CD-ROM disk 1122, or reading data from other high-capacity optical media, such as a DVD, or recording data in the high-capacity optical media). A hard disk drive 1114, a magnetic disk drive 1116, and an optical disk drive 1120 may be connected to a system bus 1108 by a hard disk drive interface 1124, a magnetic disk drive interface 1126, and an optical drive interface 1128, respectively. An interface 1124 for implementing an outer mounted drive includes, for example, at least one of or both a universal serial bus (USB) and the Institute of Electrical and Electronics Engineers (IEEE) 1394 interface technology.

The drives and the computer readable media associated with the drives provide non-volatile storage of data, data structures, computer executable commands, and the like. In the case of the computer 1102, the drive and the medium correspond to the storage of random data in an appropriate digital form. In the description of the computer readable media, the HDD, the portable magnetic disk, and the portable optical media, such as a CD, or a DVD, are mentioned, but those skilled in the art will well appreciate that other types of computer readable media, such as a zip drive, a magnetic cassette, a flash memory card, and a cartridge, may also be used in the illustrative operation environment, and the predetermined medium may include computer executable commands for performing the methods of the present disclosure.

A plurality of program modules including an operation system 1130, one or more application programs 1132, other program modules 1134, and program data 1136 may be stored in the drive and the RAM 1112. An entirety or a part of the operation system, the application, the module, and/or data may also be cached in the RAM 1112. It will be well appreciated that the present disclosure may be implemented by several commercially usable operation systems or a combination of operation systems.

A user may input a command and information to the computer 1102 through one or more wired/wireless input devices, for example, a keyboard 1138 and a pointing device, such as a mouse 1140. Other input devices (not illustrated) may be a microphone, an IR remote controller, a joystick, a game pad, a stylus pen, a touch screen, and the like. The foregoing and other input devices are frequently connected to the processing device 1104 through an input device interface 1142 connected to the system bus 1108, but may be connected by other interfaces, such as a parallel port, an IEEE 1394 serial port, a game port, a USB port, an IR interface, and other interfaces.

A monitor 1144 or other types of display devices are also connected to the system bus 1108 through an interface, such as a video adaptor 1146. In addition to the monitor 1144, the computer generally includes other peripheral output devices (not illustrated), such as a speaker and a printer.

The computer 1102 may be operated in a networked environment by using a logical connection to one or more remote computers, such as remote computer(s) 1148, through wired and/or wireless communication. The remote computer(s) 1148 may be a work station, a computing device computer, a router, a personal computer, a portable computer, a microprocessor-based entertainment device, a peer device, and other general network nodes, and generally includes some or an entirety of the constituent elements described for the computer 1102, but only a memory storage device 1150 is illustrated for simplicity. The illustrated logical connection includes a wired/wireless connection to a local area network (LAN) 1152 and/or a larger network, for example, a wide area network (WAN) 1154. The LAN and WAN networking environments are general in an office and a company, and make an enterprise-wide computer network, such as an Intranet, easy, and all of the LAN and WAN networking environments may be connected to a worldwide computer network, for example, the Internet.

When the computer 1102 is used in the LAN networking environment, the computer 1102 is connected to the local network 1152 through a wired and/or wireless communication network interface or an adaptor 1156. The adaptor 1156 may make wired or wireless communication to the LAN 1152 easy, and the LAN 1152 also includes a wireless access point installed therein for the communication with the wireless adaptor 1156. When the computer 1102 is used in the WAN networking environment, the computer 1102 may include a modem 1158, is connected to a communication computing device on a WAN 1154, or includes other means setting communication through the WAN 1154 via the Internet. The modem 1158, which may be an embedded or outer-mounted and wired or wireless device, is connected to the system bus 1108 through a serial port interface 1142. In the networked environment, the program modules described for the computer 1102 or some of the program modules may be stored in a remote memory/storage device 1150. The illustrated network connection is illustrative, and those skilled in the art will appreciate well that other means setting a communication link between the computers may be used.

The computer 1102 performs an operation of communicating with a predetermined wireless device or entity, for example, a printer, a scanner, a desktop and/or portable computer, a portable data assistant (PDA), a communication satellite, predetermined equipment or place related to a wirelessly detectable tag, and a telephone, which is disposed by wireless communication and is operated. The operation includes a wireless fidelity (Wi-Fi) and Bluetooth wireless technology at least. Accordingly, the communication may have a pre-defined structure, such as a network in the related art, or may be simply ad hoc communication between at least two devices.

The Wi-Fi enables a connection to the Internet and the like even without a wire. The Wi-Fi is a wireless technology, such as a cellular phone, which enables the device, for example, the computer, to transmit and receive data indoors and outdoors, that is, in any place within a communication range of a base station. A Wi-Fi network uses a wireless technology, which is called IEEE 802.11 (a, b, g, etc.) for providing a safe, reliable, and high-rate wireless connection. The Wi-Fi may be used for connecting the computer to the computer, the Internet, and the wired network (IEEE 802.3 or Ethernet is used). The Wi-Fi network may be operated at, for example, a data rate of 11 Mbps (802.11a) or 54 Mbps (802.11b) in an unauthorized 2.4 and 5 GHz wireless band, or may be operated in a product including both bands (dual bands).

Those skilled in the art may appreciate that information and signals may be expressed by using predetermined various different technologies and techniques. For example, data, indications, commands, information, signals, bits, symbols, and chips referable in the foregoing description may be expressed with voltages, currents, electromagnetic waves, magnetic fields or particles, optical fields or particles, or a predetermined combination thereof.

Those skilled in the art will appreciate that the various illustrative logical blocks, modules, processors, means, circuits, and algorithm operations described in relationship to the embodiments disclosed herein may be implemented by electronic hardware (for convenience, called "software" herein), various forms of program or design code, or a combination thereof. In order to clearly describe compatibility of the hardware and the software, various illustrative components, blocks, modules, circuits, and operations are generally illustrated above in relation to the functions of the hardware and the software. Whether the function is implemented as hardware or software depends on design limits given to a specific application or an entire system. Those skilled in the art may perform the function described by various schemes for each specific application, but it shall not be construed that the determinations of the performance depart from the scope of the present disclosure.

Various embodiments presented herein may be implemented by a method, a device, or a manufactured article using a standard programming and/or engineering technology. A term "manufactured article" includes a computer program, a carrier, or a medium accessible from a predetermined computer-readable storage device. For example, the computer-readable storage medium includes a magnetic storage device (for example, a hard disk, a floppy disk, and a magnetic strip), an optical disk (for example, a CD and a DVD), a smart card, and a flash memory device (for example, an EEPROM, a card, a stick, and a key drive), but is not limited thereto. Further, various storage media presented herein include one or more devices and/or other machine-readable media for storing information.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

The description of the presented embodiments is provided so as for those skilled in the art to use or carry out the present disclosure. Various modifications of the embodiments may be apparent to those skilled in the art, and general principles defined herein may be applied to other embodiments without departing from the scope of the present disclosure. Accordingly, the present disclosure is not limited to the embodiments suggested herein, and shall be interpreted within the broadest meaning range consistent to the principles and new characteristics presented herein.

### [Mode for Invention]

Related contents in the best mode for carrying out the present disclosure are described as above.

## Claims

1. A method for determining whether an electrocardiogram signal is readable, the method performed by a computing device, the method comprising:
assessing a quality of the electrocardiogram signal using a plurality of signal assessment modules; and
distinguishing a readable sub-interval and an unreadable sub-interval within an interval of the electrocardiogram signal based on assessment results of the plurality of signal assessment modules.

2. The method of claim 1, wherein the assessing of the quality of the electrocardiogram signal includes:
generating a signal preprocessed based on the electrocardiogram signal using at least one preprocessing scheme; and
generating a plurality of assessment results for the electrocardiogram signal by utilizing the preprocessed signal and the plurality of signal assessment modules.

3. The method of claim 1, wherein the plurality of respective signal assessment modules include different noise reading functions.

4. The method of claim 3, wherein each of the plurality of signal assessment modules identifies a readable candidate sub-interval and an unreadable candidate sub-interval based on a predetermined threshold within the interval of the electrocardiogram signal.

5. The method of claim 4, wherein the threshold is determined in response to a type of noise reading function.

6. The method of claim 4, wherein the distinguishing of the readable sub-interval and the unreadable sub-interval within the interval of the electrocardiogram signal based on the assessment results of the plurality of signal assessment modules includes:
identifying the readable sub-interval based on identifying an overlapping interval of a plurality of readable candidate sub-intervals identified by the plurality of signal assessment modules.

7. The method of claim 4, wherein the plurality of signal assessment modules include at least one of:
a first type of signal assessment module configured to identify the readable candidate sub-interval or the unreadable candidate sub-interval based on a continuous assessment value for the electrocardiogram signal; or
a second type of signal assessment module configured to identify the readable candidate sub-interval or the unreadable candidate sub-interval based on a predetermined segment-unit assessment value for the electrocardiogram signal.

8. The method of claim 7, wherein the second type of signal assessment module calculates the predetermined segment-unit assessment value using an assessment value for a base assessment-unit, when the base assessment-unit of the second type of signal assessment module is larger than the predetermined segment-unit.

9. The method of claim 8, wherein when the base assessment-unit of the second type of signal assessment module is larger than the predetermined segment-unit, the second type of signal assessment module performs:
an operation of obtaining a plurality of assessment values for base assessment-units based on a plurality of assessment start points arranged at an interval of the predetermined segment-unit; and
an operation of calculating the predetermined segment-unit assessment value by calculating a representative value of the plurality of assessment values for the base assessment-units at each interval of the predetermined segment-unit.

10. The method of claim 1, further comprising at least one of:
inputting an entire interval or the readable sub-interval of the electrocardiogram signal into a disease analysis model when a longest interval of readable sub-intervals is equal to or larger than a predetermined length; or
determining re-measurement for the electrocardiogram signal when the longest interval of the readable sub-intervals is smaller than the predetermined length.

11. A method for determining whether an electrocardiogram signal is re-measured, the method performed by a computing device, the method comprising:
distinguishing a readable sub-interval and an unreadable sub-interval within an interval of an electrocardiogram signal; and
determining whether the electrocardiogram signal is re-measured based on the distinguished readable sub-interval.

12. A computer program stored in a non-transitory computer-readable storage medium, wherein when the computer program is executed by one or more processors, the computer program allows the one or more processors to perform following operations for determining whether an electrocardiogram signal is readable, the operations comprising:
an operation of assessing a quality of the electrocardiogram signal using a plurality of signal assessment modules; and
an operation of distinguishing a readable sub-interval and an unreadable sub-interval within an interval of the electrocardiogram signal based on assessment results of the plurality of signal assessment modules.

13. A computing device comprising:
at least one processor; and
a memory,
wherein the at least one processor is configured to:
assess a quality of an electrocardiogram signal using a plurality of signal assessment modules, and
distinguish a readable sub-interval and an unreadable sub-interval within an interval of the electrocardiogram signal based on assessment results of the plurality of signal assessment modules.
